# EUROPÄISCHE PATENTSCHRIFT

(11) **EP 0 771 189 B1**
(45) Veröffentlichungstag und Bekanntmachung des Hinweises auf die Patenterteilung: **16.08.2001**
(21) Anmeldenummer: 95924299.1
(22) Anmeldetag: 21.06.1995
(51) Int. Cl.: A61K 9/00, A61K 9/14

(54) **FORMULIERUNG ZUR INHALATIVEN APPLIKATION**
INHALATION COMPOSITION
COMPOSITION POUR INHALATION

(30) Priorität: 16.07.1994 DE 4425255
(43) Veröffentlichungstag der Anmeldung: 07.05.1997
(73) Patentinhaber: ARZNEIMITTELWERK DRESDEN GmbH, FACHBEREICH PATENTE, D-01435 Radebeul (DE)
(72) Erfinder: SARLIKIOTIS, Werner, 60316 Frankfurt (DE); DE BOER, Anne H., NL-9204 AG Drachten (NL)
(74) Vertreter: Decker, Karl-Ludwig
(86) Internationale Anmeldenummer: EP9502392
(87) Internationale Veröffentlichungsnummer: WO9602231

(56) Entgegenhaltungen:
- WO-A-91/11179
- WO-A-95/11666
- DE-A- 2 851 489

## Beschreibung

Die vorliegende Erfindung betrifft eine Arzneimittelformulierung zur inhalativen Applikation, wobei der mikronisierte Wirkstoff oder das mikronisierte Wirkstoffgemisch auf einen Träger aufgetragen werden, ohne daß Bindemittel verwendet werden.

Wirkstoffe, die inhalativ appliziert werden, müssen, um topische oder auch systemische Wirkung zu zeigen, tief in die Lunge penetrieren. Um dies zu erreichen, müssen die Teilchen des Wirkstoffes einen Durchmesser, der etwa 5 µm-10 µm nicht überschreitet, aufweisen. Außerdem wird der Wirkstoff oder das Wirkstoffgemisch mit Hilfe einer speziell angefertigten Einrichtung,einem Inhalator, dem Patienten appliziert. Dabei muß der Wirkstoff erstens entweder vordosiert,zum Beispiel in Kapseln oder Blister, oder in größerer Menge im Inhalator bevorratet werden, um dann durch den Einatmungsprozess des Patienten aus einer Meßeinrichtung entnommen und mit Dispergiereinrichtung, zum Beispiel einer Wirbelkammer, in die feinen Primärpartikel redispergiert, mit dem Atemzug mitgerissen und so der Lunge zugänglich gemacht zu werden.

Die hierzu verwendeten, feinstkörnigen Wirkstoffe zeigen aufgrund der Partikelgröße eine hohe spezifische Oberfläche der Partikel und einer daraus resultierenden Kräfteverteilung sehr ausgeprägte adhäsive und kohäsive Eigenschaften, was wiederum zur Folge hat, daß die verfahrenstechnische Verarbeitung solcher Pulver auf Schwierigkeiten stößt. Solche verfahrenstechnischen Schritte sind die Mischung der Wirkstoffe bei Wirkstoffgemischen, die Lagerung und der Transport der Pulver, die Befüllung von Kapseln, Blistern oder Inhalatoren sowie die Dosierung der therapeutischen Mengen.

Die in der Pharmazie üblichen Agglomerationsverfahren, z.B. Granulation, können nicht eingesetzt werden, weil dadurch die Partikel so stark miteinander verbunden werden, daß keine lungengängigen Wirkstoffteilchen mehr vorhanden sind oder generiert werden können. Außerdem können derzeit die meisten der pharmazeutisch gängigen Hilfsstoffe keine Anwendung bei inhalativen Arzneiformen finden, da das toxikologische Verhalten dieser Stoffe bei pulmonaler Gabe noch weitgehend unbekannt ist.

Um die erwähnten Probleme zu lösen, wurde beispielsweise in EP 0 398 631 vorgeschlagen, den Wirkstoff bis zu einem mittleren Partikeldurchmesser von 5 µm bis 10 µm zu vermahlen und dann entweder mit einem festen, pharmazeutisch üblichen Träger zu vermischen, wobei dieser einen mittleren Partikeldurchmesser von 30 µm bis 80 µm aufweist, oder aber runde Agglomerate der Wirkstoffpartikel herzustellen (sog. Weichpellets), wobei diese während der Inhalation wieder in die Primärpartikel zerfallen. Ein Verfahren zur Herstellung solcher Weichpellets ist ebenso beschrieben (GB 1,569,612 und GB 1,520,247 ). Dabei muß der Feuchtigkeitsgehalt des Wirkstoffes vor der Herstellung der Weichpellets eingestellt werden. Die Weichpellets können, wie beispielsweise in DE 25 35 258 und GB 1,520,247 beschrieben in Kapseln abgefüllt werden. In vitro Versuche zeigten, daß bei der Entleerung dieser Kapseln mit Hilfe eines Inhalators mindestens 50% des abgefüllten Materials entleert wurden.

Diese nach den oben aufgeführten Vorschriften hergestellten Weichpellets weisen aber eine Dispergierrate (= Anteil der lungengängigen Wirkstoffteilchen nach der Entleerung, bezogen auf die in der Kapsel abgefüllten Menge) auf, die noch nicht befriedigen kann.
Bei einem Volumenstrom von 60 1/min wurden für kommerziell erhältliche Systeme im Kaskadenimpektor (four-Stage-Liquid Impinger) Dispergierraten von 13,8-29,5% der Nominal-Dosis gefunden.

Bei einem anderen Verfahren (DE 22 29 981) wird der Wirkstoff mit einem pharmazeutisch verwendbaren, wasserlöslichen Träger vermischt, wobei dieser Träger eine Teilchengröße von 80 µm bis 150 µm aufweist.
Nachteilig sind hier die schlechten Fließeigenschaften der Formulierung.

DE 41 40 689 beschreibt Inhalationspulver, die aus einem physiologisch unbedenklichen Hilfsstoff mit einer mittleren Teilchengröße von ca 20 µm und einer zweiten Komponente Hilfsstoff mit kleineren Teilchen mit etwa 10 µm Teilchengröße besteht.
Diese Mischung ist in Kapseln abfüllbar und wird mit Geräten inhaliert, die in DE 33 45 722 beschrieben sind. Auch hier sind die schlechten Fließeigenschaften von Nachteil.

EP 258 356 beschreibt Mikropartikel für Inhalationszwecke, die aus einem Konglomerat aus Hilfsstoffen, beispielsweise Lactose, Xylit und Mannit mit der Größe zwischen 30 µm und 150 µm bestehen. Problematisch ist bei diesem Verfahren der relativ komplexe Herstellungsgang, um die Hilfsstoffkonglomerate mit einer bestimmten Partikelgröße herzustellen.

DE 28 51 489 A1 beschreibt eine Formulierung, bestehend aus Beclomethasondipropionat, dessen Partikel zu 90 Gew.-% kleiner als 10 µm sind und aus einem Pulverträger, der aus Teilchen mit einer wirksamen Größe von 90 Gew.-% kleiner 400 µm und von mindestens 50 Gew.-% größer 30 µm besteht. Diese Formulierung kann neben dem Wirkstoff Beclomethasondipropionat noch einen Bronchdilatator von vorzugsweise der gleichen Teilchengröße wie Beclomethasondipropionat enthalten. Als Bronchodilatatoren werden Orciprenalin, Terbutalin oder Salbutamol bezeichnet.

Die Umsetzung zu einem pharmazeutischen Präparat erfolgt durch einfaches Vermischen.

WO 91/11179 A1 beschreibt die Anwendung von Trägermaterialien wie Lactose, Polysacchariden und weiteren. Diese Trägermaterialien weisen beschreibungsgemäß eine Partikelgröße von 5.0 - 1000 µm und eine Oberflächenrauheit von weniger als 1,75 auf. Ein einfaches Vermischen des Trägermaterials mit einem pharmazeutischen Wirkstoff führt zu der Pulverzubereitung.

WO 95/11666 A1 beschreibt ein Verfahren zur Herstellung von Trägerpartikeln, bei dem mit Hilfe einer mechanischen Behandlung auf der Oberfläche von Partikeln Teilchen abgetragen und ggf. wieder aufgetragen werden. Die so behandelten Trägerpartikel können durch Mischen mit Wirkstoffpulver beladen werden und zeigen eine verbesserte Redispergierung von auf der Oberfläche haftenden Wirkstoffpartikeln.

Es besteht also die Aufgabe, zum Zwecke der Inhalation ein Pulver zu entwickeln, das einfach herzustellen ist, keine aufwendigen Inprozeß-Kontrollen des Feuchtigkeitsgehalts der Wirkstoffe und/oder Hilfsstoffe erfordert und einen hohen Grad an Redispergierung aufweist. Weiter soll die Formulierung befriedigende Fließeigenschaften aufweisen und im Inhalator leicht in lungengängige Partikel zerlegbar sein. Bei gleichen Bedingungen sollten mindestens 40 % redispergierbar werden.

Die Lösung der gestellten Aufgabe ergibt sich aus den Ansprüchen.

Bei der erfindungsgemäßen Formulierung kann auf eine Konditionierung, beispielsweise auf zusätzliche Reinigungsprozesse, des eingesetzten Trägermaterials verzichtet werden.
Die Teilchen des Trägers sind kommerziell erhältlich oder können durch Fraktionierung (Sieb) in einer bestimmten Korngröße beziehungsweise Korngrößenbereich erhalten werden.

Die Bestimmung der Teilchengröße der Trägerteilchen erfolgte durch Ausmessen von rasterelektronenmikroskopischen Aufnahmen und/oder durch Siebanalyse. Die Bestimmung der Teilchengröße der Wirkstoffpartikel erfolgte durch Ausmessen von rasterelektronenmikroskopischen Aufnahmen und/oder durch Laserbeugungsspektrometrie.

Diese Pulverformulierung ist einfach und wirtschaftlich herstellbar und besitzt sowohl im Vergleich zu dem unbehandelten Wirkstoffpulver als auch zu den Weichpellets erheblich bessere Fließeigenschaften. Dies zeigen die Ergebnisse in Tabelle 1. Eine geringere Schütthöhe bedeutet bessere Fließeigenschaften der Formulierung.
Je ähnlicher Schütt- und Stampfvolumen sind, desto besser sind die Fließeigenschaften. Aber auch die Entleerung und anschließende Redispergierung ist im Vergleich zu den bisher bekannten Formulierungen (Mischungen, Weichpellets nach GB 1 569 612 oder GB 1 520 247 oder unbehandeltes Wirkstoffpulver) besser, d.h. die Rückstände im Inhalator sind geringer und die Ausbeute an lungengängigen Teilchen höher.

Die Formulierung kann verschiedene Wirkstoffe enthalten beispielsweise Analgetika, Antiallergika, Antibiotika, Anticholinergika, Antihistaminika, antiinflammatorisch wirkende Substanzen, Antipyretika, Kortikoide, Steroide, Antitussiva, Bronchodilatatoren, Diuretika, Enzyme, Herz-Kreislauf wirksame Substanzen, Hormone, Proteine und Peptide. Beispiele für Analgetika sind Codein, Diamorphin, Dihydromorphin, Ergotamin, Fentanyl und Morphin; Beispiele für Antiallergika sind Cromoglicinsäure und Nedocromil; Beispiele für Antibiotika sind Cephalosporine, Fusafungin, Neomycin, Penicilline, Pentamidin, Streptomycin, Sulfonamide und Tetracycline; Beispiele für Anticholinergika sind Atropin, Atropinmethonitrat, Ipratropiumbromid, Oxitropiumbromid und Trospiumchlorid; Beispiele für Antihistaminika sind Azelastin, Flezelastin und Methapyrilen; Beispiele für antiinflammatorisch wirksame Substanzen sind Beclometason, Budesonid, Dexamethason, Flunisolid, Fluticason, Tipredane und Triamcinolon; Beispiele für Antitussiva sind Narcotin und Noscapin; Beispiele für Bronchodilatatoren sind Bambuterol, Bitolterol, Carbuterol, Clenbuterol, Ephedrin, Epinephrin Formoterol, Fenoterol, Hexoprenalin, Ibuterol, Isoprenalin, Isoproterenol, Metaproterenol, Orciprenalin, Phenylephrin, Phenylpropanolamin, Pirbuterol, Procaterol, Reproterol, Rimiterol, Salbutamol, Salmeterol, Sulfonterol, Terbutalin und Tolobuterol; Beispiele für Diuretika sind Amilorid und Furosemid; ein Beispiel für Enzyme ist Trypsin; Beispiele für Herz-Kreislauf wirksame Substanzen sind Diltiazem und Nitroglycerin; Beispiele für Hormone sind Cortison, Hydrocortison und Prednisolon; Beispiele für Proteine und Peptide sind Cyclosporine, Cetrorelix, Glucagon und Insulin. Weitere Wirkstoffe, die eingesetzt werden können, sind Adrenochrom, Colchicin, Heparin, Scopolamin. Die beispielhaft angeführten Wirkstoffe können als freie Basen oder Säuren oder als pharmazeutisch verträgliche Salze eingesetzt werden. Als Gegenionen können beispielsweise physiologisch verträgliche Erdalkali- oder Alkalimetalle oder Amine sowie beispielsweise Acetat, Benzolsulfonat, Benzoat, Hydrogencarbonat, Hydrogentartrat, Bromid, Chlorid, Iodid, Carbonat, Citrat, Fumarat, Malat, Maleat, Gluconat, Lactat, Pamoat und Sulphat eingesetzt werden. Es können auch Ester eingesetzt werden, beispielsweise Acetat, Acetonid, Propionat, Dipropionat, Valerat.

Die erfindungsgemäße Formulierung kann auch aus einem Gemisch mehrerer fein gemahlener Wirkstoffe bestehen, beispielsweise aus Natriumcromoglicat und Reproterolhydrochlorid. Wie bereits beschrieben, sollten 100 % der Wirkstoffteilchen kleiner als 10 µm sein, vorzugsweise im Bereich von 1 µm bis 5 µm liegen.

Das Verhältnis von Wirkstoff zum Trägermaterial hängt von den eingesetzten Stoffen ab. Es hat sich anhand der Beispiele gezeigt, daß die Verwendung von 5 bis 80 Gewichtsprozent des Wirkstoffes auf 20 bis 90 Gewichtsprozent des Trägers, vorzugsweise 30 bis 70 Gewichtsprozent des Wirkstoffes auf 30 bis 70 Gewichtsprozent des Trägerstoffes befriedigende Ergebnisse liefert.

Zusätzlich zum Wirkstoff und Träger können die Formulierungen auch andere Bestandteile, wie Geschmackskorrigenzien, zum Beispiel Saccharin oder Pfefferminzaroma, enthalten.Die Komponenten können zum Beispiel 10-20 Gewichts% bezogen auf den Wirkstoff beziehungsweise auf das Wirkstoffgemisch betragen.

Die Herstellung der Formulierung erfolgt durch Mischen der Bestandteile in einem geeignetem Mischer, beispielsweise Taumelmischer, Rotationsmischer, Schnellmischer oder fluidisierende Mischer. Als Taumelmischer kommt beispielsweise der Turbula-Mischer, W.A. Bachofen AG, Basel, CH; als Schnellmischer der Diosna-Mischer, Dierks und Söhne, Osnabrück, BRD, in Frage.
Dabei werden die Bestandteile dem Mischer zugeführt und so lange gemischt, bis die Trägerkristalle mit dem feinen Wirkstoff oder Wirkstoffgemisch überzogen sind, wobei der Feinanteil allmählich verschwindet und runde, überzogene Teilchen entstehen.

Aber auch andere Verfahren wie Wirbelschicht- oder
Vibrations-Verfahren können zur Herstellung der erfindungsgemäßen Pulverformulierungen herangezogen werden. Bei diesen Verfahren werden die Trägerstoffteilchen in einem Behälter in rotierender Bewegung versetzt. Dadurch können sich die Wirkstoffteilchen darauf ablagern und so die erfindungsgemäße Formulierung bilden.

Um die Vorteile der erfindungsgemäßen Formulierung im Vergleich zu der Mischung der beiden Wirkstoffe und den Weichpellets gemäß den Schriften GB 1,569,612 und GB 1,520,247 zu demonstrieren, wurden diese Formulierungen hergestellt und einige physikalische Meßgrößen bestimmt.

Die Herstellung der Mischung der beiden Wirkstoffe erfolgte mit Hilfe eines Taumelmischers (Turbula-Mischer; W.A. Bachofen AG Basel). Die Weichpellets wurden hergestellt, indem die feinen Wirkstoffe in das Bodengefäß eines Siebturmes zur Teilchengrößenanalyse (Retsch, BRD) gebracht wurden, und das Gefäß so lange Vibrationen ausgesetzt wurde, bis runde Wirkstoffagglomerate entstanden.

Folgende Tabellen zeigen einige vergleichende Meßergebnisse.

### Versuch 1

Wirkstoffmischung: Zwei Gewichtsteile Dinatrium Cromoglicat und ein Gewichtsteil Reproterolhydrochlorid.

| | Kernagglomerate | Mischung | Weichpellets |
|---|---|---|---|
| Schüttvolumen (ml/g) | 2,2 | 7,2 | 3,8 |
| Stampfvolumen (20x) (ml/g) | 2 | 5 | 3 |
| Hausner-Faktor | 1,10 | 1,44 | 1,27 |
| Schütthöhe (mm) | 24 | 35 | 29 |
| Redispergierung (%) bei 60 l/min/Volumen-strom | 50 | 40 | 35 |
| Redispergierung (%) bei 30 l/min/Volumen-strom | 30 | 10 | 15 |

Die Weichpellets wurden gemäß den Vorschriften GB 1,569,612 und GB 1,520,247 erhalten.

### Versuch 2

Wirkstoffmischung: Drei Gewichtsteile Dinatrium Cromoglicat und zwei Gewichtsteile Reproterolhydrochlorid.

| | Kernagglomerate | Mischung | Weichpellets |
|---|---|---|---|
| Schüttvolumen (ml/g) | 2 | 7,2 | 3,8 |
| Stampfvolumen (20x) (ml/g) | 1,9 | 5 | 3 |
| Hausner-Faktor | 1,05 | 1,44 | 1,27 |
| Schütthöhe (mm) | 23 | 35 | 29 |
| Fließwinkel ( ° ) | 48 | 59 | 54 |

Schütt- und Stampfvolumen wurden nach bekannten Verfahren ermittelt.

100 g Formulierung werden in einen Meßzylinder vorsichtig eingeschüttet. Das abgelesene Volumen stellt das Schüttvolumen dar. Der gefüllte Meßzylinder wird an ein Stampfvolumeter angebracht. Es werden 20 Stampfungen durchgeführt. Das abgelesene Volumen stellt das Stampfvolumen dar (siehe auch Voigt R., Lehrbuch der pharmazeutischen Technologie, Verlag Chemie, 5. Auflage, Seite 148).
Der Hausner-Faktor ist das Verhältnis aus Schütt- und Stampfvolumen.

Die Schütthöhe wurde mit Hilfe eines Zylinders mit Durchmesser 42 mm bestimmt, wobei so viel Pulver langsam aufgeschüttet wurde bis ein maximal hohes Haufwerk entstand, dessen Höhe gemessen wurde. Die Redispergierung wurde mit Hilfe eines Inhalators und eines Kaskadenimpaktors bestimmt, indem die Anteile in Prozent bezogen auf die Einwaage bestimmt wurden, die sich auf die zweite bis vierte Kaskade abgelagert hatten. Dieser Versuch wurde mit zwei verschiedenen Volumenströmen durchgeführt.

### Beispiel 1

266.8 g mikronisiertes Dinatrium Cromoglicat und 133.2 g mikronisiertes Reproterolhydrochlorid werden durch ein Sieb der Maschenweite 0,125 mm gesiebt und anschließend in einen Diosna Mischer PWC Dierks und Söhne, Osnabrück BRD) gegeben. Dazu werden 600.0 g handelsübliche Lactose mit einer Korngrößenverteilung 100% < 800 µm, 12% - 35% < 400 µm und max 7% < 200 µm gegeben. Anschließend wird 30 min gemischt. Die so entstandenen Kernagglomerate sind gut fließfähig und können in einen Inhalator abgefüllt werden. Die Eigenschaften dieser Kernagglomerate können dem Versuch 1 (Seite 8) entnommen werden.

### Beispiel 2

3000 g mikronisiertes Dinatrium Cromoglicat und 200,0 g mikronisiertes Reproterolhydrochlorid werden durch ein Sieb der Maschenweite 0,125 mm gesiebt und anschließend in einen Taumelmischer (Turbula-Mischer; W.A. Bachofen AG Basel)gegeben. Dazu werden 500,0 g handelsübliche Lactose mit einer Korngrößenverteilung 100% < 800 µm, 12% - 35% < 400 µm und max 7% < 200 µm gegeben. Anschließend wird 30 min gemischt. Die so entstandenen Kernagglomerate sind gut fließfähig und können in einen Inhalator abgefüllt werden. Die Eigenschaften dieser Kernagglomerate können dem Versuch 2 (Seite 9) entnommen werden.

### Beispiel 3

30 g mikronisiertes Budesonid werden mit Hilfe eines 0,125-mm-Siebes gesiebt und anschließend in einen Taumelmischer (Tubula-Mischer; W.A. Bachofen AG, Basel) gegeben. Dazu werden 270 g handelsübliche Lactose mit einer Korngrößenverteilung 100% K 800µm, 12%-35% L 400 µm und maximal 7% L 200µm gegeben. Anschließend wird 45 min gemischt. Die so entstandenen Kernagglomerate sind gut fließfähig und können in einen Inhalator, eine Patrone oder Blister abgefüllt werden.

### Beispiel 4

100 g mikronisiertes Salbutamol werden mit Hilfe eines 0,125-mm-Siebes gesiebt und anschließend in einen Taumelmischer (Tubula-Mischer; W.A. Bachofen AG, Basel) gegeben. Dazu werden 300 g handelsübliche Lactose mit einer Korngrößenverteilung 100% K 800µm, 12%-35% L 400 µm und maximal 7% L 200µm gegeben. Anschließend wird 45 min gemischt. Die so entstandenen Kernagglomerate sind gut fließfähig und können in einen Inhalator, eine Patrone oder Blister abgefüllt werden.

### Beispiel 5

20 g mikronisiertes Beclometason-17,21-dipropionat werden mit Hilfe eines 0,125-mm-Siebes gesiebt und anschließend in einen Taumelmischer (Turbula-Mischer; W.A. Bachofen AG, Basel) gegeben. Dazu werden 380 g handelsübliche Lactose mit einer Korngrößenverteilung 100 % < 800 µm, 12 % - 35 % < 400 µm und maximal 7 % < 200 µm gegeben. Anschließend wird 45 min gemischt. Die so entstandenen Kernagglomerate sind gut fließfähig und können in einen Inhalator, eine Patrone oder Blister abgefüllt werden.

### Beispiel 6

20 g mikronisiertes Ipratropiumbromid werden mit Hilfe eines 0,125-mm-Siebes gesiebt und anschließend in einen Taumelmischer (Turbula-Mischer; W.A. Bachofen AG, Basel) gegeben. Dazu werden 380 g handelsübliche Lactose mit einer Korngrößenverteilung 100 % < 800 µm, 12 % - 35 % < 400 µm und maximal 7 % < 200 µm gegeben. Anschließend wird 45 min gemischt. Die so entstandenen Kernagglomerate sind gut fließfähig und können in einen Inhalator, eine Patrone oder Blister abgefüllt werden.

## Patentansprüche

1. Mischung, dadurch gekennzeichnet, daß ein Wirkstoff oder ein Wirkstoffgemisch mit einer mittleren Teilchengröße von 0,01 µm bis 10 µm mit kommerziell verwendeter Lactose als Träger mit mittlerer Teilchengröße zwischen 600 µm bis kleiner 1000 µm vermischt wird.

2. Mischung, nach Anspruch 1 dadurch gekennzeichnet, daß ein Wirkstoff oder ein Wirkstoffgemisch mit einer mittleren Teilchengröße von 1 µm bis 5 µm verwendet wird.

3. Verfahren zur Herstellung einer Formulierung gemäß Ansprüchen 1 oder 2 zur Herstellung von Arzneimitteln zur Inhalation.

4. Verfahren zur Herstellung einer Formulierung zur-Herstellung von Arzneimitteln gemäß Anspruch 1 und 2,
dadurch gekennzeichnet, daß die Trägerteilchen von den Wirkstoffteilchen umhüllt sind.

5. Verwendung der Formulierung zur Herstellung von Arzneimitteln gemäß einem der Ansprüche 1 - 2, dadurch gekennzeichnet, daß diese sich aus 5 bis 80 Gewichtsprozent des Wirkstoffes oder des Wirkstoffgemisches und 20 bis 90 Gewichtsprozent des Trägers, vorzugsweise aus 30 bis 70 Gewichtsprozent des Wirkstoffes und 30 bis 70 Gewichtsprozent des Trägerstoffes zusammensetzt.

6. Verwendung der Formulierung zur Herstellung von Arzneimitteln gemäß einem der Ansprüche 1 - 2, dadurch gekennzeichnet, daß neben Wirkstoff oder dem Wirkstoffgemisch und Trägerstoff oder Trägergemisch auch weitere physiologisch akzeptable Hilfsstoffe enthalten sind.

7. Arzneimittelformulierung gemäß einem der Ansprüche 1-2, dadurch gekennzeichnet, daß als Wirkstoffmischung eine Mischung aus Reproterol und dem Dinatriumsalz der Cromoglicinsäure verwendet wird.

8. Arzneimittelformulierung gemäß einem der Ansprüche 1-2, dadurch gekennzeichnet, daß als Wirkstoff Budesonid verwendet wird.

9. Arzneimittelformulierung gemäß einem der Ansprüche 1-2, dadurch gekennzeichnet, daß als Wirkstoff Salbutamol verwendet wird.

10. Arzneimittelformulierung gemäß einem der Ansprüche 1-2, dadurch gekennzeichnet, daß als Wirkstoff Cetrorelix verwendet wird.

11. Arzneimittelformulierung gemäß einem der Ansprüche 1-2, dadurch gekennzeichnet, daß als Wirkstoff Beclometason verwendet wird.

12. Arzneimittelformulierung gemäß einem der Ansprüche 1-2, dadurch gekennzeichnet, daß als Wirkstoff Ipratropiumbromid verwendet wird.

## Claims

1. Mixture, characterized in that an active compound or an active compound mixture having a mean particle size of 0.01 µm to 10 µm is mixed with commercially used lactose as the excipient having a mean particle size of between 600 *µ*m to [sic] less than 1000 µm.

2. Mixture according to Claim 1, characterized in that an active compound or an active compound mixture having a mean particle size of 1 µm to 5 µm is used.

3. Process for the preparation of a formulation according to Claims 1 or 2 for the production of medicaments for inhalation.

4. Process for the preparation of a formulation for the production of medicaments according to Claim 1 and 2, characterized in that the excipient particles are covered by the active compound particles.

5. Use of the formulation for the production of medicaments according to one of Claims 1 - 2, characterized in that this is composed of 5 to 80 percent by weight of the active compound or of the active compound mixture and 20 to 90 percent by weight of the excipient, preferably of 30 to 70 percent by weight of the active compound and 30 to 70 percent by weight of the excipient.

6. Use of the formulation for the production of medicaments according to one of Claims 1 - 2, characterized in that, besides active compound or the active compound mixture and excipient or excipient mixture, further physiologically acceptable auxiliaries are also contained.

7. Pharmaceutical formulation according to one of Claims 1 - 2, characterized in that the active compound mixture used is a mixture of reproterol and the disodium salt of cromoglycic acid.

8. Pharmaceutical formulation according to one of Claims 1 - 2, characterized in that the active compound used is budesonide.

9. Pharmaceutical formulation according to one of Claims 1 - 2, characterized in that the active compound used is salbutamol.

10. Pharmaceutical formulation according to one of Claims 1 - 2, characterized in that the active compound used is cetrorelix.

11. Pharmaceutical formulation according to one of Claims 1 - 2, characterized in that the active compound used is beclometasone [sic].

12. Pharmaceutical formulation according to one of Claims 1 - 2, characterized in that the active compound used is ipratropium bromide.

## Revendications

1. Formulation,
caractérisée en ce qu'
on mélange un principe actif ou un mélange de principes actifs ayant une taille moyenne de particules allant de 0,01 à 10 µm avec du lactose utilisé dans le commerce comme support ayant une taille moyenne de particules comprise entre 600 et inférieure à 1000 µm.

2. Formulation selon la revendication 1,
caractérisée en ce qu'
on utilise un principe actif ou un mélange de principes actifs ayant une taille moyenne de particules allant de 1 à 5 µm.

3. Procédé de préparation d'une formulation selon les revendications 1 ou 2, en vue de la production de médicaments pour inhalation.

4. Procédé de préparation d'une formulation en vue de la production de médicaments selon la revendication 1 ou 2,
caractérisé en ce que
les particules de support sont enrobées par les particules de principe actif.

5. Utilisation de la formulation en vue de la production de médicaments selon l'une des revendications 1 ou 2,
caractérisée en ce que
celle-ci se compose de 5 à 80 pour-cent en poids de principe actif ou du mélange de principes actifs et de 20 à 90 pour-cent en poids de support, de préférence de 30 à 70 pour-cent en poids de principe actif et de 30 à 70 % en poids de la substance de support.

6. Utilisation de la formulation en vue de la production de médicaments selon l'une des revendications 1 ou 2,
caractérisée en ce qu'
à côté du principe actif ou du mélange de principes actifs et de la substance de support ou du mélange de supports également d'autres substances auxiliaires physiologiquement acceptables sont contenues.

7. Formulation de médicament selon l'une des revendications 1 ou 2,
caractérisée en ce que
comme mélange de principes actifs on utilise un mélange à base de reprotérol et de sel disodique d'acide cromoglycique.

8. Formulation de médicament selon l'une des revendications 1 ou 2,
caractérisée en ce qu'
on utilise comme principe actif du budésonide.

9. Formulation de médicament selon l'une des revendications 1 ou 2,
caractérisée en ce qu'
on utilise comme principe actif du salbutamol.

10. Formulation de médicament selon l'une des revendications 1 ou 2,
caractérisée en ce qu'
on utilise comme principe actif du cétrorélix.

11. Formulation de médicament selon l'une des revendications 1 ou 2,
caractérisée en ce qu'
on utilise comme principe actif de la beclométasone.

12. Formulation de médicament selon l'une des revendications 1 ou 2,
caractérisée en ce qu'
on utilise comme principe actif du bromure d'ipratropium.
